# EUROPEAN PATENT SPECIFICATION

(11) **EP 2 572 591 B1**
(45) Date of publication and mention of the grant of the patent: **12.07.2017**
(21) Application number: 11783459.8
(22) Date of filing: 13.05.2011
(51) Int. Cl.: C12N 1/16, C12P 13/06, A23L 27/00, A23L 27/21, A23L 27/24

(54) **ALANINE-RICH SEASONING COMPOSITION**
ALANINREICHE GEWÜRZZUSAMMENSETZUNG
COMPOSITION D'ASSAISONNEMENT RICHE EN ALANINE

(30) Priority: 17.05.2010 JP 2010113614
(43) Date of publication of application: 27.03.2013
(73) Proprietor: Asahi Group Holdings, Ltd., Tokyo 130-8602 (JP)
(72) Inventor: ODANI, Tetsuji, Moriya-shi Ibaraki 302-0106 (JP); TADAMI, Hideyo, Moriya-shi Ibaraki 302-0106 (JP); OKUTOMI, Keiichi, Tokyo 130-8602 (JP); SHIBUYA, Ichiro, Moriya-shi Ibaraki 302-0106 (JP)
(74) Representative: Müller, Christian Stefan Gerd
(86) International application number: PCT/JP2011/061074
(87) International publication number: WO 2011/145525

(56) References cited:
- EP-A1- 2 351 829
- WO-A1-2006/025295
- WO-A1-2010/058558
- JP-A- 1 108 977
- JP-A- 10 327 802
- JP-A- 60 133 892
- JP-A- 2006 067 813
- JP-B- 39 016 342
- JP-B- 54 031 076
- US-A1- 2008 138 467
- US-B1- 6 344 231

## Description

### Technical Field

The present invention relates to an alanine-rich seasoning composition, and particularly relates to an alanine-rich seasoning composition which is a yeast extract.

### Background Art

A composition containing amino acids is well known as a seasoning for foods and drinks. For example, a protein hydrolysate is a sweetener produced by hydrolyzing mainly collagen and gelatin. The protein hydrolysate contains proline, glycine and alanine which are constituent amino acids of collagen and gelatin in large amounts. These free amino acids exhibiting a sweet taste are referred to as sweet amino acids.

The protein hydrolysate is widely used for enhancing the sweet taste of processed foods such as a sauce, a dressing and the like. However, when the protein hydrolysate is produced by a hydrochloric acid decomposition method, monochloropropanediol (MCP) and dichloropropanol (DCP) are generated as by-products. These substances are suspected of being toxic to the human body, and a method for reducing the substances to provide a safe seasoning is employed, but a safer seasoning is desired.

On the other hand, the yeast contains a large amount of amino acids in fungus bodies. An amino acid contained in the yeast is extracted as an extract from fungus bodies by extraction with a solvent such as hot water or the like. A yeast extract is produced from a naturally-derived yeast, and is a seasoning that is highly safe. The yeast contains mainly glutamic acid, and the yeast extract has an excellent function of enhancing the umami taste.

However, the yeast extract has a low content of sweet amino acids such as proline, glycine, alanine and the like, and has a poor function of enhancing a sweet taste. For example, Patent Document 1 describes a yeast extract, the proline content of which is increased for enhancing a sweet taste. The yeast extract in Patent Document 1 is used for enhancing the sweet taste of processed foods such as a sauce, a dressing and the like as an alternative to an animal protein hydrolysate.

It is described that the yeast extract described in Patent Document 1 contains free proline, one of sweet amino acids, in an amount of 8% or more of the free amino acid composition, and achieves an effect of enhancing a sweet taste. However, the content of alanine, also one of sweet amino acids, is still low as compared to an animal protein hydrolysate. Thus, the yeast extract described in Patent Document 1 cannot be said to be adequate in terms of either a sweet taste or a first taste, and is somewhat incomplete for use as an alternative to an animal protein hydrolysate.

JP 10-327802 A discloses a yeast extract useful as a taste enhancer having increased contents of alanine, glutamic acid and histidine.

### Prior Art Document

### Patent Document

Patent Document 1: WO 2008/081519

### Summary of the Invention

### Problem to be Solved by the Invention

The present invention solves the conventional problem described above, and an object thereof is to provide a seasoning composition which is highly safe to the human body, has excellent sweet taste and first taste, and can be used as an alternative to an animal protein hydrolysate without causing any problem.

### Means for Solving the Problem

The present invention provides a yeast extract according to claim 1.

In a certain aspect, the yeast is Saccharomyces cerevisiae, Candida utilis or a variant thereof having mycological properties same as those thereof.

In a certain aspect, the conditions advantageous for production of alanine are to adjust pH of a liquid culture medium of a yeast in a stationary phase to 7.5 to 11, and further carry out culture.

In a certain aspect, a protein hydrolysate prepared using a hydrochloric acid decomposition method is not included.

In a certain aspect, the yeast extract is a seasoning composition.

### Effects of the Invention

A seasoning composition of the present invention is highly safe to the human body, has excellent sweet taste and first taste, and can be used as an alternative for an animal protein hydrolysate without causing any problem.

### Brief Description of the Drawings

Fig. 1 is a graph showing a curve of an increase in the number of fungi versus culture time in Example 1.
Fig. 2 is a graph showing a curve of an increase in weight of dry yeast fungus bodies versus culture time in Example 1.
Fig. 3 is a graph showing a change in pH of a liquid culture medium versus culture time in Example 1.

### Mode for Carrying Out the Invention

### Yeast

A seasoning composition of the present invention consists of a yeast extract as a source of an amino acid that is a taste component. A yeast is a unicellular fungus. Specific examples of the yeast include genus Saccharomyces fungi, genus Schizosaccharomyces fungi, genus Pichia fungi, genus Candida fungi, genus Kluyveromyces fungi, genus Williopsis fungi, genus Debaryomyces fungi, genus Galactomyces fungi, genus Torulaspora fungi, genus Rhodotorula fungi, genus Yarrowia fungi, genus Zygosaccharomyces fungi and the like. A variant obtained by a normal method can also be used.

Among them, edible and preferable yeasts are Candida tropicalis, Candida lypolitica, Candida utilis, Candida sake, Saccharomyces cerevisiae and the like, and more preferable are Saccharomyces cerevisiae and Candida utilis that are generally used.

### Culture of Yeast

The yeast is cultured in a liquid culture medium under conditions advantageous for the yeast to produce alanine. The conditions advantageous for the yeast to produce alanine are culture conditions in which pH of the liquid culture medium is 7.5 or more and less than 11 in a stationary phase of growth of the yeast.

The liquid culture medium is a medium containing a nutrient source for the yeast to grow. Generally, an aqueous solution containing a carbon source, a nitrogen source, an inorganic salt and the like is used as the liquid culture medium. As the carbon source of the liquid culture medium, one or more selected from the group consisting of glucose, sucrose, acetic acid, ethanol, a molasses, a sulfite pulp waste liquor and the like are used. As the nitrogen source, one or more selected from the group consisting of inorganic salts such as urea, ammonia, ammonium sulfate, ammonium chloride, ammonium phosphate and the like, nitrogen-containing organic substances such as a corn steep liquor (CSL), casein, a yeast extract, peptone and the like, and the like are used.

Further, a phosphoric acid component, a potassium component and a magnesium component may be added to the liquid culture medium. Specific examples of these components include normal industrial raw materials such as superphosphate of lime, ammonium phosphate, potassium chloride, potassium hydroxide, magnesium sulfate, magnesium chloride and the like. In addition, an inorganic salt including a zinc ion, a copper ion, a manganese ion, an iron ion or the like, a vitamin, a nucleic acid associated substance and the like may be added.

The culture form of the yeast may be any of batch culture, feeding culture and continuous culture. When culture is carried out in an industrial scale, the culture form of feeding culture or continuous culture is normally employed.

In a logarithmic growth phase, the yeast may be cultured under general culture conditions. For example, temperature of the liquid culture medium is 20 to 40°C, preferably 25 to 35°C, and pH of the liquid culture medium is 3.5 to 7.5, preferably 4.0 to 6.0. Aerobic conditions are preferable. It is preferable to carry out culture with aeration and stirring. The amount of aeration and conditions for stirring can be appropriately determined in consideration of volume and time of culture and initial concentration of fungi. For example, aeration can be carried out at about 0.2 to 2 V.V.M (volume per volume per minutes), and stirring can be carried out at about 50 to 800 rpm.

In a stationary phase of growth of the yeast, the liquid culture medium is adjusted to alkaline pH. The range of pH that is preferable as alkaline pH is pH 7.5 to 11, more preferably pH 7.5 to 10.0. For the method for adjusting pH, for example, an appropriate amount of alkaline component may be added. It is preferable to adjust pH of the liquid culture medium just after entrance to a stationary phase from the logarithmic growth phase. This is because alanine concentration in the yeast is adequately increased. In addition, this is because time required for a process of culturing the yeast is reduced.

Examples of the alkaline component include inorganic alkalis such as aqueous ammonia (NH₄OH), an ammonia gas, sodium hydroxide, potassium hydroxide, calcium hydroxide, magnesium hydroxide and the like; alkaline bases such as sodium carbonate, potassium carbonate and the like; organic alkalis such as urea and the like; and the like. Among the alkaline components described above, aqueous ammonia, an ammonia gas and urea are preferable.

pH of the liquid culture medium may be adjusted, for example, by adding an alkaline component at the time of entrance to a stationary phase of growth of the yeast. Alternatively, an organic alkali such as urea may be added in the culture medium beforehand, so that pH is spontaneously adjusted with elapse of culture time. The amount of alkaline component added to the culture medium is generally about 0.5 to 5% by weight based on the liquid culture medium.

Culture temperature after adjustment of pH of the liquid culture medium, and other culture conditions may follow general yeast culture conditions. For example, culture temperature is 20 to 40°C, preferably 25 to 35°C.

### Yeast Extract

If the yeast is cultured by the method described above, the content of sweet amino acids in yeast fungus bodies is increased. Among sweet amino acids, those that are increased are alanine and proline, especially alanine. As a result, a yeast which plentifully contains sweet amino acids, especially alanine and proline, in fungus bodies is produced.

By extracting amino acids in the fungus body from the produced yeast, a yeast extract having a high content of sweet amino acids is obtained. Examples of a method for extracting an amino acid in the fungus body of the yeast include an autolysis method, an enzyme decomposition method, an acid decomposition method, an alkali extraction method, a hot water extraction method and the like. Among them, the hot water extraction method is highly safe and preferable as a method for extracting an amino acid in yeast fungus bodies for the purpose of producing a seasoning.

The yeast extract produced from the yeast described above has a high content of alanine and proline, especially alanine. The content of free alanine is 20% by weight or more, preferably 20 to 50% by weight, more preferably 25 to 50% by weight, especially preferably 30 to 45% by weight based on the total amount of free amino acids contained in the yeast extract. If the content of free alanine is less than 20% by weight, the first taste and sweet taste of the yeast extract, especially the first taste, become inadequate. The content of free proline is 5% by weight or more, preferably 10 to 35% by weight, more preferably 15 to 35% by weight, especially preferably 15 to 30% by weight based on the total amount of free amino acids contained in the yeast extract. If the content of free proline is less than 3% by weight, the sweet taste of the yeast extract becomes inadequate.

The content of free alanine is 4.0% by weight or more, preferably 6 to 30% by weight, more preferably 10 to 30% by weight, especially preferably 12 to 25% by weight based on the yeast extract. The content of free proline is 1.0% by weight or more, preferably 1.5 to 15% by weight, more preferably 2 to 15% by weight, especially preferably 3 to 13% by weight based on the yeast extract. The total of free alanine and free proline is 13% by weight or more, preferably 15 to 30% by weight, more preferably 17 to 30% by weight, especially preferably 19 to 28% by weight based on the yeast extract.

The content of free alanine is 2.0% by weight or more, preferably 2.5 to 10% by weight, more preferably 3.0 to 10% by weight, especially preferably 3.0 to 8% by weight based on dry yeast fungus bodies. The content of free proline is 0.5% by weight or more, preferably 1.0 to 5% by weight, more preferably 1.5 to 5% by weight, especially preferably 1.7 to 4% by weight based on dry yeast fungus bodies. The total of free alanine and free proline is 2.5% by weight or more, preferably 3 to 10% by weight, more preferably 4 to 10% by weight, especially preferably 5 to 8% by weight based on dry yeast fungus bodies.

If the yeast is cultured by the method described above, the content of umami amino acids in yeast fungus bodies is increased. The umami amino acids include glutamic acid. As a result, the yeast extract of the present invention has an increased content of alanine, proline and glutamic acid. However, since the amount of glutamic acid contained in the yeast extract is an appropriate amount, the action of enhancing the sweet taste by alanine and proline is not adversely affected.

The content of free glutamic acid is 59% by weight or less, preferably 50% by weight or less, more preferably 25 to 47% by weight, especially preferably 29 to 44% by weight based on the total amount of free amino acids contained in the yeast extract. If the content of free glutamic acid is more than 60% by weight, the umami taste of the yeast extract becomes strong, and the first taste and the sweet taste become inadequate.

The total amount of free alanine, free proline and free glutamic acid contained in the yeast extract is 14% by weight or more, preferably 20% by weight or more, more preferably 22 to 45% by weight, especially preferably 26 to 42% by weight. If the total amount described above is less than 14% by weight, the first taste and the sweet taste of the yeast extract become inadequate.

The ratio of the total amount of the free alanine and free proline to the amount of the free glutamic acid is 1.1 to 1.7. If the ratio described above is less than 0.5, the umami taste of the yeast extract becomes strong, and the first taste and the sweet taste become inadequate.

A free amino acid that is a component of the yeast extract, which is extracted from yeast fungus bodies, is particularly called an endogenous free amino acid. For example, free alanine that is a component of the yeast extract is called endogenous free alanine. Free proline that is a component of the yeast extract is called endogenous free proline. Free glutamic acid that is a component of the yeast extract is called endogenous free glutamic acid.

The yeast extract of the present invention contains a large amount of sweet amino acids, especially alanine and proline. The yeast extract contains an appropriate amount of glutamic acid that is an umami amino acid. As a result, the yeast extract of the present invention has a refreshing and neat taste and leaves no heavy aftertaste compared to a conventional yeast extract. In addition, the yeast extract exhibits a sweet taste close to that of an animal protein hydrolysate.

### Seasoning Composition

The yeast extract of the present invention is highly safe to the human body, and can be used as a seasoning for foods and drinks.

### Use

The yeast extract and the seasoning composition of the present invention (hereinafter referred to as a yeast extract and the like of the present invention) can be liquid, paste-like, powdery or granular. In addition, they can be used together with other seasonings and additives acceptable as foods and drinks.

The yeast extract and the like of the present invention can be added to various kinds of foods and drinks (including health foods). They can also be added to pharmaceuticals, cosmetics and pet foods besides foods and drinks.

The yeast extract and the like of the present invention have sweet taste quality comparable to that of an animal protein decomposition product, and therefore can be used as an alternative to an animal protein decomposition product in foods and drinks for which the animal protein decomposition product is suitable (for example, as an umami and sweet taste improving agent and an alternative to a protein hydrolysate).

The yeast extract and the like of the present invention can be utilized for meat/fish/vegetable dishes, and specific examples of foods and drinks include white sauce, meat sauce, demi-glace sauce, tomato sauce, curry, stew, potage soup, minestrone, sauces (Worcester sauce, moderately thick sauce, thick sauce, Tonkatsu sauce, Okonomiyaki sauce, fried soba sauce, Takoyaki sauce and the like), noodle soups (Chinese noodle soup, buckwheat noodle soup, Japanese wheat noodle and the like), casserole soups (Oden soup and the like), miso soup, chop suey, chukadon, Chinese-style fried rice, jiao-zi, shao mai, baozi, snack foods, dressings, seasoned powders for sprinkling over rice, gravy sauces (sauce for barbecued meat), food boiled down in soy and the like.

The yeast extract and the like of the present invention can be used for frozen foods, retort pouch foods and instant foods.

The present invention will be described further specifically with examples below, but the present invention is not limited thereto. "%" in examples is based on weight unless otherwise specified.

### Examples

### Example 1

### Preparation of Yeast Extract R

### <1> Preculture

In 350 ml of a liquid culture medium having the following composition was inoculated 300 ml of yeast Saccharomyces cerevisiae ABS5 strain, and the yeast was cultured at 30°C for 24 hours with shaking at a speed of 160 rpm using Bio Shaker manufactured by TAITEC CORPORATION to obtain a yeast preculture solution.

**[Table 1]**

| (Liquid Medium Composition) | |
|---|---|
| Molasses (sugar content 36%) | 8% |
| Urea | 0.6% |
| Ammonium sulfate ((NH₄)₂SO₄) | 0.16% |
| Diammonium hydrogen phosphate ((NH₄)₂HPO₄) | 0.08% |
| Milli Q water | Balance |

### <2> Main Culture

To 2 L of a liquid culture medium having the following composition was added 600 ml of the obtained preculture solution, the resulting mixture was aerated at a speed of 3 L/minute, and stirred at a speed of 600 rpm, pH of the culture solution was controlled to 5.0 using 10% aqueous ammonia, and culture was performed at 30°C while feeding 800 ml of molasses having a sugar content of 36% at a speed of 0.8 ml/minute. The yeast started logarithmic growth.

**[Table 2]**

| (Liquid Medium Composition) | |
|---|---|
| Ammonium chloride (NH₄Cl) | 0.18% |
| Diammonium hydrogen phosphate ((NH₄)₂HPO₄) | 0.04% |

### <3> pH shift

Just after entrance to a stationary phase of growth of the yeast, 100 ml of 10% aqueous ammonia was added to the culture solution to adjust pH of the culture solution to 9.0 (hereinafter referred to as a "pH shift"), and culture was further continued. Culture was completed 48 hours after main culture was started.

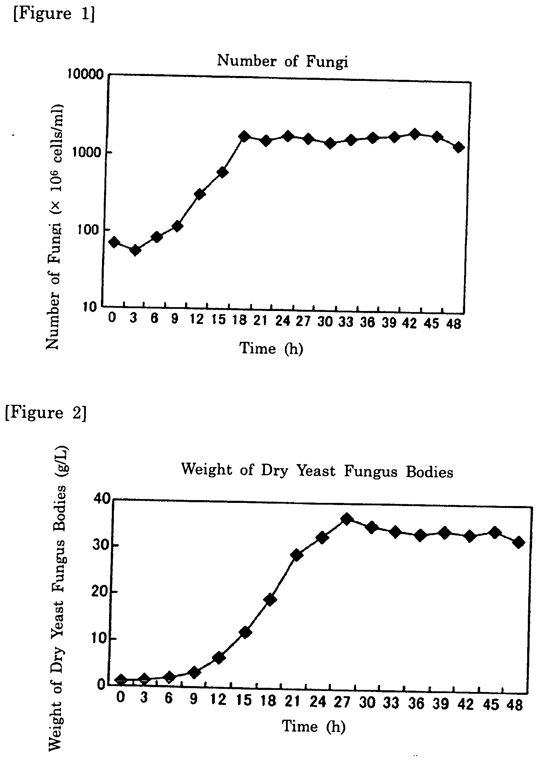

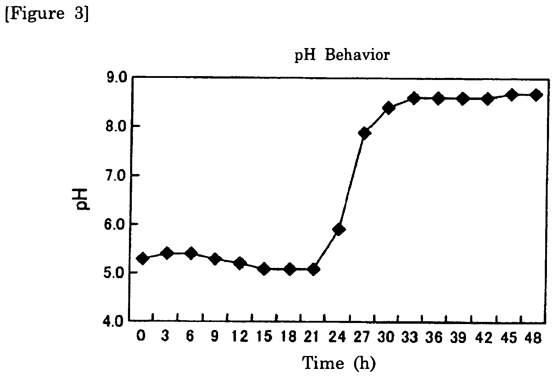

Fig. 1 shows a curve of an increase in the number of fungi versus culture time. Fig. 2 shows a curve of an increase in weight of dry yeast fungus bodies versus culture time. Fig. 3 shows a change in pH of the culture solution versus culture time.

As shown in Fig. 1, an increase in the number of fungi (× 10⁶ cells/ml) reached a stationary state 18 hours after culture, showing entrance to a stationary phase of growth. Weight of dry yeast fungus bodies (g/L) was also in an approximately stationary state 24 hours after culture, showing a stationary phase of growth. Measurement of pH of the culture solution showed that pH shifted to alkaline pH after entrance to the stationary phase of growth as shown in Fig. 3.

### <4> Yeast Extract

About 18 ml of a culture solution of the yeast was subjected to centrifugal separation (3000 g, 20°C, 5 min), about 1.5 ml of separated solid was transferred to an Eppendorf tube, and the tube was transferred to a block heater, and heated at 80°C for 30 minutes. Thereafter, the heated product was subjected to centrifugal separation (6000 g, 4°C, 5 min), and the obtained supernatant was collected as a yeast extract R.

### <5> Measurement of Alanine Content

The amount of alanine contained in the yeast extract R was measured. The content of free alanine was 42.7% based on the total amount of free amino acids. The content of free alanine was measured by an AccQ-Tag Ultra labeling method using "Acquity UPLC" Analyzer manufactured by Waters Corporation (US). In the measurement method, free alanine in a sample can be selectively quantified. A calibration curve was prepared using Amino Acids Mixture Standard Solution, Type H (manufactured by Wako Pure Chemical Industries, Ltd.). The content of alanine in the yeast extract was 16.6%, and the content of alanine per dry yeast fungus body was 5.0%.

### <6> Measurement of Proline Content

The amount of proline contained in the yeast extract R was measured. The content of free proline was 15.7% based on the total amount of free amino acids. The content of proline in the yeast extract was 6.1%, and the content of proline per dry yeast fungus body was 1.9%.

### Examples 2 to 30

For a yeast extract obtained in the same manner as in Example 1 except that the type of a strain was different, the contents of amino acids (alanine, proline) in the extract were measured. Compositions in free amino acids were calculated from the total amount of free amino acids, and a ratio to dry yeast fungus bodies was calculated from weight of dry yeast fungus bodies (Examples 2 to 4).

For a yeast extract obtained as a result of conducting an additional test in the same manner as in Example 1, the contents of amino acids (alanine, proline) in the extract were measured. Compositions in free amino acids were calculated from the total amount of free amino acids, and a ratio to dry yeast fungus bodies was calculated from weight of dry yeast fungus bodies (Examples 5 to 30).

As shown in Table 3, an alanine and proline-rich yeast extract was obtained even when the type of a strain was different. An alanine and proline-rich yeast extract was obtained even when the additional test was repeatedly conducted.

**[Table 3]**

| | Strain | Composition in total free amino acids (%) | | Content (%) in yeast extract | | Ratio (%) to dry yeast fungus bodies | |
|---|---|---|---|---|---|---|---|
| | | Ala | Pro | Ala | Pro | Ala | Pro |
| Example 2 | Saccharomyces cerevisiae ABS1 | 35.6 | 8.6 | 22.3 | 5.4 | 5.3 | 1.3 |
| Example 3 | Saccharomyces cerevisiae ABS2 | 36.2 | 9.5 | 7.4 | 1.9 | 1.4 | 0.4 |
| Example 4 | Candida utilis ABC1 | 23.7 | - | 6.0 | - | 2.5 | - |
| Example 5 | Saccharomyces cerevisiae ABS5 | 28.2 | 30.3 | 8.6 | 9.2 | 3.1 | 3.3 |
| Example 6 | Saccharomyces cerevisiae ABS5 | 22.0 | 28.7 | 7.7 | 10.1 | 2.5 | 3.2 |
| Example 7 | Saccharomyces cerevisiae ABS5 | 29.8 | 17.0 | 12.3 | 7.0 | 3.9 | 2.2 |
| Example 8 | Saccharomyces cerevisiae ABS5 | 28.6 | 17.3 | 12.5 | 7.6 | 4.0 | 2.4 |
| Example 9 | Saccharomyces cerevisiae ABS5 | 19.0 | 18.0 | 8.0 | 7.6 | 2.7 | 2.6 |
| Example 10 | Saccharomyces cerevisiae ABS5 | 28.0 | 17.0 | 11.9 | 7.2 | 3.3 | 2.0 |
| Example 11 | Saccharomyces cerevisiae ABS5 | 32.2 | 22.3 | 12.2 | 8.5 | 3.2 | 2.2 |
| Example 12 | Saccharomyces cerevisiae ABS5 | 27.0 | 22.3 | 12.3 | 10.1 | 3.5 | 2.9 |
| Example 13 | Saccharomyces cerevisiae ABS5 | 28.8 | 19.0 | 12.1 | 8.0 | 3.9 | 2.6 |
| Example 14 | Saccharomyces cerevisiae ABS5 | 27.3 | 22.8 | 12.1 | 10.1 | 3.8 | 3.1 |
| Example 15 | Saccharomyces cerevisiae ABS5 | 27.1 | 20.9 | 12.8 | 9.9 | 4.3 | 3.4 |
| Example 16 | Saccharomyces cerevisiae ABS5 | 29.6 | 22.5 | 12.4 | 9.4 | 3.7 | 2.8 |
| Example 17 | Saccharomyces cerevisiae ABS5 | 31.5 | 21.8 | 13.1 | 9.0 | 3.5 | 2.4 |
| Example 18 | Saccharomyces cerevisiae ABS5 | 32.9 | 21.0 | 14.3 | 9.1 | 4.0 | 2.5 |
| Example 19 | Saccharomyces cerevisiae ABS5 | 32.3 | 22.0 | 12.3 | 8.4 | 3.6 | 2.4 |
| Example 20 | Saccharomyces cerevisiae ABS5 | 42.7 | 15.7 | 16.6 | 6.1 | 5.0 | 1.9 |
| Example 21 | Saccharomyces cerevisiae ABS5 | 28.3 | 15.9 | 8.4 | 4.7 | 2.5 | 1.4 |
| Example 22 | Saccharomyces cerevisiae ABS5 | 30.2 | 19.6 | 14.2 | 9.2 | 4.5 | 2.9 |
| Example 23 | Saccharomyces cerevisiae ABS5 | 31.5 | 25.8 | 13.9 | 11.4 | 3.6 | 3.0 |
| Example 24 | Saccharomyces cerevisiae ABS5 | 36.6 | 19.0 | 15.8 | 8.2 | 4.7 | 2.5 |
| Example 25 | Saccharomyces cerevisiae ABS5 | 36.9 | 17.5 | 13.9 | 6.6 | 4.0 | 1.9 |
| Example 26 | Saccharomyces cerevisiae ABS5 | 40.1 | 5.1 | 12.1 | 1.5 | 3.8 | 0.5 |
| Example 28 | Saccharomyces cerevisiae ABS5 | 32.5 | 27.7 | 10.8 | 9.2 | 3.3 | 2.8 |
| Example 29 | Saccharomyces cerevisiae ABS5 | 41.2 | 9.3 | 16.6 | 3.7 | 4.6 | 1.0 |
| Example 30 | Saccharomyces cerevisiae ABS5 | 41.5 | 8.9 | 15.9 | 3.4 | 4.7 | 1.0 |

### Example 31

### Preparation of Yeast Extracts C, P and Q

### <1> Control Yeast Extract C

For the control, Meast Powder N, a standard type yeast extract of ASAHI FOOD & HEALTHCARE CO., LTD was used as a yeast extract C. The content of free alanine in the yeast extract C was 10.2% based on the total amount of free amino acids, and the content of free proline was 2.3% based on the total amount of free amino acids. In the yeast extract C, the measurable contents of various kinds of free amino acids were comparable to those in the yeast extract of Example 1 except that the contents of free alanine and free proline were low.

### <2> Yeast Extracts P and Q

Then, 150 ml of the yeast extract R and 350 ml of the yeast extract C were mixed to prepare a yeast extract P. The content of free alanine in the yeast extract P was 20.4% based on the total amount of free amino acids, and the content of free proline was 6.1% based on the total amount of free amino acids.

Then, 250 ml of the yeast extract R and 250 ml of the yeast extract C were mixed to prepare a yeast extract Q. The content of free alanine in the yeast extract Q was 27.1% based on the total amount of free amino acids, and the content of free proline was 8.8% based on the total amount of free amino acids.

### Reference Example

Examples of animal protein hydrolysates include CH Kyowa (Kirin Kyowa Foods Company, Limited). It is widely known that the animal protein hydrolysate contains alanine, glycine and proline that are main components of collagen and gelatin in large amounts, and among them, alanine and glycine are concerned with the first taste. CH Kyowa contained 12.2% by weight of free alanine based on the total amount of free amino acids and 14.5% by weight of free proline based on the total amount of free amino acids. Free glycine was also contained in a large amount, and the total of free alanine and free glycine constituted 38.1% of free amino acids.

### Sensory Test

Ten panelists trained for evaluation of yeast extracts conducted sensory tests. For yeast extracts of examples and a comparative example, a hot aqueous solution having a solid content of 1% was prepared, and salt was added so as to constitute 9% of the solid content, and a sensory test was conducted. The sensory test had a score of 1 to 7 points presented with sweet taste intensity of the yeast extract C fixed to 4 points and a score of 1 to 7 points presented with first taste intensity of the yeast extract C fixed to 4 points, and the scores were represented by an average. The results are shown in Table 4.

**[Table 4]**

| Test section | Amino acid content^{a} | | Sweet taste intensity | First taste intensity | Sweet taste quality | Comments |
|---|---|---|---|---|---|---|
| | Alanine | Proline | | | | |
| Yeast Extract C | 10.2 | 2.3 | 4.0 | 4.0 | | |
| Yeast Extract P | 20.4 | 6.1 | 4.3 | 4.3 | Slightly sweet | |
| Yeast Extract Q | 27.1 | 8.8 | 5.3 | 5.3 | First sweet | Balanced |
| Yeast Extract R | 42.7 | 15.7 | 6.7 | 6.0 | First-moderately sweet | Sustained |
| Reference Example 1 | 12.2 (38.1)^{b} | 14.5 | 4.7 | 6.3 | | Seafood taste |

| | | | | | | |
|---|---|---|---|---|---|---|
| a) % by weight based on the total amount of free amino acids b) Reference 1 shows % by weight of alanine and % by weight of (alanine + glycine). | | | | | | |

When the content of alanine was 20% or more, an increase in sweet taste intensity and first taste intensity was observed. The yeast extract R had a value close to the ratio of alanine + glycine (38.1%) in the animal protein hydrolysate in Reference Example 1, and was evaluated as having comparable first taste intensity in the sensory evaluation test. Sweet taste quality was closer to that of the animal protein hydrolysate.

### Examples 32 to 47

A yeast extract was obtained in the same manner as in Example 1. The contents of amino acids (alanine, proline and glutamic acid) in the obtained yeast extract were measured. Compositions in free amino acids were calculated from the total amount of free amino acids, a ratio to dry yeast fungus bodies was calculated from weight of dry yeast fungus bodies, and the total amount of alanine, proline and glutamic acid, and a ratio of the total amount of alanine and proline to the amount of glutamic acid were calculated.

As shown in Table 5, a yeast extract containing a large amount of alanine and proline and an appropriate amount of glutamic acid was obtained.

**[Table 5]**

| | Strain | Composition in total free amino acids (%) | | | Content (%) in yeast extract | | | | Ratio (%) to dry yeast fungus bodies | | | |
|---|---|---|---|---|---|---|---|---|---|---|---|---|
| | | Ala | Pro | Glu | Ala | Pro | Glu | A+P+Glu¹⁾ | Ala | Pro | Glu | A+P/Glu²⁾ |
| Example 32 | Saccharomyces cerevisiae ABS5 | 22 | 28.7 | 33.2 | 7.7 | 10.1 | 11.7 | 29.5 | 2.5 | 3.2 | 3.8 | 1.52 |
| Example 33 | Saccharomyces cerevisiae ABS5 | 29.8 | 17 | 32 | 12.3 | 7 | 13.2 | 32.5 | 3.9 | 2.2 | 4.2 | 1.46 |
| Example 34 | Saccharomyces cerevisiae ABS5 | 28.6 | 17.3 | 29.1 | 12.5 | 7.6 | 12.8 | 32.9 | 4 | 2.4 | 4.1 | 1.57 |
| Example 35 | Saccharomyces cerevisiae ABS5 | 19 | 18 | 32.6 | 8 | 7.6 | 13.8 | 29.4 | 2.7 | 2.6 | 4.7 | 1.13 |
| Example 36 | Saccharomyces cerevisiae ABS5 | 28 | 17 | 32.5 | 11.9 | 7.2 | 13.8 | 32.9 | 3.3 | 2 | 3.8 | 1.38 |
| Example 37 | Saccharomyces cerevisiae ABS5 | 28.8 | 19 | 34.1 | 12.1 | 8 | 14.3 | 34.4 | 3.9 | 2.6 | 4.6 | 1.41 |
| Example 38 | Saccharomyces cerevisiae ABS5 | 27.3 | 22.8 | 29.6 | 12.1 | 10.1 | 13.1 | 35.3 | 3.8 | 3.1 | 4.1 | 1.69 |
| Example 39 | Saccharomyces cerevisiae ABS5 | 27.1 | 20.9 | 30.1 | 12.8 | 9.9 | 14.2 | 36.9 | 4.3 | 3.4 | 4.8 | 1.60 |
| Example 40 | Saccharomyces cerevisiae ABS5 | 29.6 | 22.5 | 33.7 | 12.4 | 9.4 | 14.1 | 35.9 | 3.7 | 2.8 | 4.2 | 1.55 |
| Example 41 | Saccharomyces cerevisiae ABS5 | 32.3 | 22 | 33.9 | 12.3 | 8.4 | 12.9 | 33.6 | 3.6 | 2.4 | 3.8 | 1.60 |
| Example 42 | Saccharomyces cerevisiae ABS5 | 28.3 | 15.9 | 43.5 | 8.4 | 4.7 | 12.9 | 26 | 2.5 | 1.4 | 3.8 | 1.02 |
| Example 43 | Saccharomyces cerevisiae ABS5 | 30.2 | 19.6 | 38 | 14.2 | 9.2 | 17.8 | 41.2 | 4.5 | 2.9 | 5.6 | 1.31 |
| Example 44 | Saccharomyces cerevisiae ABS5 | 36.6 | 19 | 33.8 | 15.8 | 8.2 | 14.6 | 38.6 | 4.7 | 2.5 | 4.3 | 1.64 |
| Example 45 | Saccharomyces cerevisiae ABS5 | 36.9 | 17.5 | 36.9 | 13.9 | 6.6 | 13.9 | 34.4 | 4 | 1.9 | 4.0 | 1.47 |
| Example 46 | Saccharomyces cerevisiae ABS5 | 41.2 | 9.3 | 40.4 | 16.6 | 3.7 | 16.2 | 36.5 | 4.6 | 1 | 4.5 | 1.25 |
| Example 47 | Saccharomyces cerevisiae ABS5 | 41.5 | 8.9 | 41.6 | 15.9 | 3.4 | 16 | 35.3 | 4.7 | 1 | 4.7 | 1.21 |

| | | | | | | | | | | | | |
|---|---|---|---|---|---|---|---|---|---|---|---|---|
| 1) Total amount of free alanine, free proline and free glutamic acid 2) Ratio of total amount of free alanine and free proline to amount of free glutamic acid | | | | | | | | | | | | |

## Claims

1. A yeast extract extracted from yeast fungus bodies cultured under conditions advantageous for production of alanine, wherein
the yeast extract contains 20% by weight or more of free alanine based on free amino acids,
the yeast extract contains 5% by weight or more of free proline based on free amino acids,
the yeast extract contains 59% by weight or less of free glutamic acid based on free amino acids,
the total of the free alanine, the free proline and the free glutamic acid is 14% by weight or more based on the yeast extract, and
the ratio of the total of the free alanine and the free proline to the free glutamic acid is 1.1 to 1.7.

2. The yeast extract according to claim 1, wherein the yeast is Saccharomyces cerevisiae, Candida utilis or a variant thereof having mycological properties same as those thereof.

3. The yeast extract according to claim 1 or 2, wherein the conditions advantageous for production of alanine are to adjust pH of a liquid culture medium of a yeast in a stationary phase to 7.5 to 11, and further carry out culture.

4. The yeast extract according to any of claims 1 to 3, wherein the yeast extract is a seasoning composition.

## Patentansprüche

1. Hefeextrakt, der aus Hefepilzkörpern extrahiert ist, die unter Bedingungen kultiviert wurden, die für eine Produktion von Alanin vorteilhaft sind, wobei
der Hefeextrakt 20% nach Gewicht oder mehr an freiem Alanin, basierend auf freien Aminosäuren, enthält,
der Hefeextrakt 5% nach Gewicht oder mehr an freiem Prolin, basierend auf freien Aminosäuren, enthält,
der Hefeextrakt 59% nach Gewicht oder weniger an freier Glutaminsäure, basierend auf freien Aminosäuren, enthält,
die Gesamtmenge des freien Alanins, des freien Prolins und der freien Glutaminsäure 14% nach Gewicht oder mehr, basierend auf dem Hefeextrakt, beträgt, und
das Verhältnis der Gesamtmenge des freien Alanins und des freien Prolins zu der freien Glutaminsäure 1,1 bis 1,7 beträgt.

2. Hefeextrakt gemäß Anspruch 1, wobei die Hefe Saccharomyces cerevisiae, Candida utilis oder eine Variante davon ist, die die gleichen mykologischen Eigenschaften wie diejenigen davon aufweist.

3. Hefeextrakt gemäß Anspruch 1 oder 2, wobei die Bedingungen, die für eine Produktion von Alanin vorteilhaft sind, das Einstellen des pH-Werts eines flüssigen Kulturmediums einer Hefe in einer stationären Phase auf 7,5 bis 11 und ein Weiterführen der Kultivierung sind.

4. Hefeextrakt gemäß einem jeglichen der Ansprüche 1 bis 3, wobei der Hefeextrakt eine Würzzusammensetzung ist.

## Revendications

1. Extrait de levure extrait des corps de champignon de levure cultivés dans des conditions avantageuses pour la production d'alanine, dans lequel
l'extrait de levure contient 20% en poids ou plus d'alanine libre sur la base des acides aminés libres,
l'extrait de levure contient 5% en poids ou plus de proline libre sur la base des acides aminés libres,
l'extrait de levure contient 59% en poids ou moins d'acide glutamique libre sur la base des acides aminés libres,
le total de l'alanine libre, la proline libre et l'acide glutamique libre est de 14% en poids ou plus sur la base de l'extrait de levure, et
le rapport du total de l'alanine libre et de la proline libre à l'acide glutamique libre est de 1,1 à 1,7.

2. Extrait de levure selon la revendication 1, dans lequel la levure est Saccharomyces cerevisiae, Candida utilis ou une de leurs variantes ayant des propriétés mycologiques semblables aux leurs.

3. Extrait de levure selon la revendication 1 ou 2, dans lequel les conditions avantageuses pour la production d'alanine sont l'ajustement du pH d'un milieu de culture liquide d'une levure dans une phase stationnaire à 7,5 à 11, et la poursuite de la culture.

4. Extrait de levure selon l'une quelconque des revendications 1 à 3, dans lequel l'extrait de levure est une composition d'assaisonnement.
